Europäisches Patentamt

**(19)** European Patent Office

Office européen des brevets

**(11)** **EP 0 524 973 B1**

**(12)** **EUROPEAN PATENT SPECIFICATION**

**(45)** Date of publication and mention
of the grant of the patent:
**08.01.1997 Bulletin 1997/02**

**(21)** Application number: **91906950.0**

**(22)** Date of filing: **22.03.1991**

**(51)** Int. Cl.[6]: **A01J 21/00**, B01J 13/00,
B01J 13/02, B32B 5/16,
B32B 9/00, A61K 7/00,
B01J 13/18

**(86)** International application number:
**PCT/US91/01868**

**(87)** International publication number:
**WO 91/15947 (31.10.1991 Gazette 1991/25)**

**(54) PREPARATION OF DISCRETE MICRODROPLETS OF AN OIL IN WATER STABILIZED BY IN SITU POLYMERIZATION OF A WATER-SOLUBLE VINYL MONOMER**

HERSTELLUNG DISKRETER MIKROTROPFEN BESTEHEND AUS EINER ÖL/WASSEREMULSION, STABILISIERT DURCH IN-SITU POLYMERISATION EINES WASSERLÖSLICHEN VINYLMONOMERS

PREPARATION DE MICROGOUTTELETTES DISCRETES D'UNE EMULSION AQUEUSE STABILISEE PAR POLYMERISATION IN SITU D'UN MONOMERE VINYLIQUE SOLUBLE DANS L'EAU

**(84)** Designated Contracting States:
**CH DE FR GB IT LI**

**(30)** Priority: **17.04.1990 US 510017**
**29.10.1990 US 604263**
**07.01.1991 US 637838**
**08.01.1991 US 638596**
**08.01.1991 US 638597**
**08.01.1991 US 638598**

**(43)** Date of publication of application:
**03.02.1993 Bulletin 1993/05**

**(73)** Proprietor: **ISP INVESTMENTS INC.**
**Wilmington, Delaware 19801 (US)**

**(72)** Inventors:
• **KOPOLOW, Stephen, L.**
**Plainsboro, NJ 08536 (US)**
• **BURLANT, William, J.**
**Wayne, NJ 07470 (US)**
• **HELIOFF, Michael, W.**
**Westfield, NJ 07090 (US)**

• **BIRES, Carmen, D.**
**Hackettstown, NJ 07840 (US)**
• **LOGIN, Robert, B.**
**Oakland, NJ 07436 (US)**
• **TAZI, Mohammed**
**Wayne, NJ 07470 (US)**
• **NEGRIN, Max**
**New York, NY 10016 (US)**
• **DANDREAUX, Gary**
**Bloomfield, NJ 07003 (US)**

**(74)** Representative: **Ford, Michael Frederick et al**
**MEWBURN ELLIS**
**York House**
**23 Kingsway**
**London WC2B 6HP (GB)**

**(56)** References cited:
EP-A- 0 055 801    EP-A- 0 111 895
EP-A- 0 214 568    EP-A- 0 270 249
US-A- 2 969 330    US-A- 3 016 308
US-A- 3 516 941    US-A- 3 763 347
US-A- 4 251 386    US-A- 4 976 961

EP 0 524 973 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.13.11/3.4

## Description

This invention relates to a method for stabilization of oils in water, and more particularly, to a method for preparing stable, discrete microdroplets of an oil in water stabilized by a water-soluble polymer solution.

The unique properties of many oils make it desirable to include them in aqueous-based compositions. For example, cosmetically-active materials such as silicone oils, fluids and gums, mineral oils, and water-insoluble organic esters such as isopropyl palmitate and isopropyl myristate, are particularly useful in cosmetic formulations for the skin and hair. In such compositions, their lubricity properties impart conditioning action for the user. However, such oils are immiscible with water which makes it very difficult to maintain a stable aqueous dispersion without rapid separation of the composition into oil and water phases. To solve the problem of providing effective dispersibility of such materials in water, it has been necessary to include a surfactant in aqueous cosmetic compositions containing cosmetically-active oils in order to maintain dispersed droplets of the oil in the aqueous solution. However, the use of surfactants increases the cost of the product and may effect the quality of the composition. In addition, even with a surfactant present, the stability of the dispersion is often not completely satisfactory.

Another approach is to form macroscopic capsules of an oil by in situ aqueous polymerization of oil soluble monomers. For example, Brynko, in US-A-2,969,330 and 2,969,331, described the preparation of pressure-rupturable capsules of a chlorinated diphenyl oil in water by dissolving styrene, an acrylate or vinyl acetate monomer in the oil, dispersing the monomer-containing oil in water with the aid of an emulsifier to form droplets, and polymerizing the monomer to form an encapsulating wall of solid polymer material around each droplet of oil.

Berg, in J. Microencapsulation (1989) 6, No. 3, 327-337, also described a process for the microencapsulation of emulsified oil droplets by in situ vinyl polymerization. However, the process was limited to the use of methyl methacrylate, an oil soluble monomer, to form a polymer shell around emulsified oil droplets of decane and hexadecane.

De Luca, in US-A-4,741,872, described the preparation of biodegradable microspheres having a three-dimensional network in which biologically active macromolecular agents were physically entrapped therein. The method involved emulsifying a vinyl derivative of a biodegradable hydrophilic polymer, a water-soluble monovinyl monomer, and a biologically active macromolecular agent, in water, and copolymerizing the vinyl compounds.

However, these and other processes have not provided a method by which cosmetically active oils, such as silicone oils, could be prepared as a stable dispersion in an aqueous medium. Nor does the prior art suggest a procedure for allowing such oils to maintain themselves in stable condition in an aqueous cosmetic formulation.

EP-A-214568 discloses an emulsion comprising a continuous aqueous phase and a discontinuous oil phase and containing an emulsifier which comprises entirely or partly at least one soap of a palmitic acid-stearic acid mixture. It is also disclosed that, in the case of cosmetic oil-water emulsions, 0.05-3.0% of at least one water soluble polymer may be added to the aqueous phase for cold stabilization.

EP-A-55081 discloses a method of thickening an aqueous system by combining said system with an aqueous dispersion of a synthetic polymer of a stated composition. The document states that water insoluble material may be present in the aqueous system. It teaches that polymerisation is carried out in an aqueous phase to form an emulsion which is then added to the aqueous system to be stabilised. This system may include water-insoluble material.

EP-A-270249 discloses a composition for fixing and setting curls in hair. The composition contains a combination of silicone and cationic organic polymer components.

EP-A-111895 discloses a skin care composition which includes an oil component and optionally also includes organic polymer.

A feature of the present invention is a process for stabilizing oils in water, preferably in the form of microdroplets, maintained discretely and for an extended period of time in an aqueous medium.

Another feature of this invention is a method for preparing an aqueous composition which includes stable, discrete microdroplets of an oil dispersed therein.

Still another feature of the present invention is a method of preparing a composition in which said microdroplets are homogeneously distributed in the composition.

Yet another feature is a method of making such stable, dispersed microdroplets by in situ polymerization of a water-soluble vinyl monomer, such as vinylpyrrolidone, in the presence of dispersed droplets of a water-insoluble oil, such as silicone oil, in water.

Among the other features of the invention is a cosmetic formulation for the care of the hair or skin, such as a conditioning hair care composition, containing stable, discrete microdroplets of a cosmetically-active oil stabilized in an aqueous solution in situ polymerized vinylpyrrolidone.

These and other features of the invention will be made apparent from the following description thereof.

<u>Abbreviations and Definitions</u>

| | |
|---|---|
| Oil - | A compound which is a water-insoluble liquid at room temperature and has an oily consistency |

| | |
|---|---|
| VP - | Vinylpyrrolidone |
| Acryl Comonomer - | A water-soluble acrylic, acrylate, acrylamide monomer, quaternized or unquaternized, e.g. a quaternized amino acrylamide |
| MAPTAC - | Methacrylamidopropyltrimethylammonium chloride |
| PVP - | Polyvinylpyrrolidone |
| DM - | Polydimethylsiloxane, Dimethicone, 100 cs($mm^2.s^{-1}$). Petrarch Chem. Co; 1000 cs, Dow Corning Corp. |
| MO - | Mineral oil |
| TBP - | Tert-butyl peroctoate, e.g. Trigonox® 21 (AKZO Chem. Co.) |
| TBPP - | t-Butylperoxy pivalate, e.g. Lupersol 11 (Atochem N.A.) |
| Cosmetically-active oil - | An oil which imparts a particularly desirable property, e.g. lubricity, to a cosmetic formulation |
| Brookfield viscosity - | Viscosity of Stabilized Oil in Water Product in cps, as measured using a RVT spindle # 3 @ 70 rpm |

What is provided herein is a method for preparing stable, discrete microdroplets of an oil in water stabilized in a polymer solution of an in situ polymerized, water-soluble vinyl monomer. The method comprises dispersing the oil in water, optionally with a surfactant, to form microdroplets, adding a water-soluble vinyl monomer, such as vinylpyrrolidone, optionally with a comonomer, such as an acryl comonomer, e.g. methacrylamidopropylammonium chloride, and polymerizing the monomer or comonomers in situ such that the oil droplets are stabilized in the resultant aqueous polymer solution.

In the preferred form of the invention, the oil is cosmetically-active, such as is characteristic of silicone oils, mineral oils and water-insoluble esters such as isopropyl myristate and isopropyl palmitate.

The invention also includes cosmetic compositions for the care of the hair or skin, including conditioning hair care compositions containing the stabilized oil droplets in dispersed form.

The conditioning hair care composition comprises (a) a stabilized silicone product obtained by in situ polymerization of a water-soluble vinyl monomer, preferably vinylpyrrolidone, in the presence of discrete microdroplets of a silicone oil in water, (b) a surfactant, and the balance being (c) water.

The hair and skin care compositions contain the dispersion in an amount of about 0.1 to 25 weight percent of the composition, preferably about 1 to 10 weight percent, of the composition.

The active material to be dispersed in an aqueous medium are oils which are water-insoluble liquids at room temperature, and preferably, are cosmetically-active, i.e. they impart a particularly desirable property such as hair conditioning and style retention to hair and skin cosmetic formulations. Such cosmetically-active oils include silicone oils, mineral oils and water-insoluble esters such as isopropyl myristate and isopropyl palmitate.

Suitable silicone oils or fluids for use in the invention may be selected from non-volatile polyalkyl siloxanes, polyaryl siloxanes, polyalkylaryl siloxanes and polyether siloxane copolymers. Such silicone oils usually are present in the composition at a level of from about 1.0% to about 18%, preferably about 2.0% to about 8.0%. Mixtures of these compounds also may be used as long as the final mixture is non-volatile and the dispersed silicone particles are insoluble in the aqueous medium. As used herein, "insoluble" requires that the oil does not substantially dissolve in water and is essentially immiscible therewith.

Non-volatile polyalkylsiloxanes include, for example, polydimethylsiloxanes (Dimethicone) with viscosities ranging from about 5-600,000 centistokes (cs) at 25°C. These siloxanes are available, for example, from the General Electric Company as the VISCASIL series and from Dow Corning as the Dow Corning 200 products. Their viscosity can be measured by the glass capillary viscometer procedure set forth in Dow Corning Corporate Test Method CTM 0004 issued July 20, 1970. Preferably, the viscosity of these siloxanes selected have a viscosity of about 100 to about 100,000 cs, and most preferably, a viscosity of up to about 15,000 cs.

Suitable non-volatile polyalkylarylsiloxanes include, for example, polymethylphenylsiloxanes having viscosities of about 15 to 65 cs at 25°C. These siloxanes are available, for example, from the General Electric as SF 1075 methylphenyl fluid or from Dow Corning as 556 Cosmetic Grade Fluid. Additionally, poly(dimethylsiloxane) (diphenylsiloxane) copolymers having a viscosity in the range of about 10 to 100,000 cs at 25°C. are useful.

These and other suitable silicones are disclosed in US-A-2,826,551, 3,964,500 and 4,364,837; and GB-A-849,433. The booklet "Silicone Compounds", which was distributed by Petrarch Systems Inc. in 1984, also describes the preparation and properties of available silicones for use in this invention.

Other suitable oils for use herein include cosmetically-active materials such as light and heavy mineral oils, and water-insoluble organic esters such as isopropyl palmitate and isopropyl myristate.

In the practice of the present invention, the oil to be dispersed is first added to water optionally with a surfactant and then subjected to agitation to produce a fine dispersion of discrete oil microdroplets throughout the aqueous medium. The mixture is agitated sufficiently so that the dispersion is stable for a period of at least 5 to 10 minutes without separating into individual layers. Conventional laboratory and high speed agitators may be used for this purpose, as for example, conventional anchor or wide-span turbine agitators.

Thereafter, a water-soluble vinyl monomer, for example, a vinylpyrrolidone monomer such as vinylpyrrolidone itself or a derivative thereof such as an alkyl vinyl pyrrolidone, is added to the mixture, along with an appropriate free radical polymerization initiator. If desired, a comonomer is added for purposes of forming a copolymer. Suitable comonomers include, among others, acryl comonomers such as dimethylaminopropyl methacrylate, dimethylaminoethyl methacrylate, methacrylamidopropyltrimethylammonium chloride, acrylamide and neutralized acrylic acid.

Suitable free radical polymerization initiators for polymerization of water-soluble vinyl monomers include such free radical catalysts as t-butylperoctoate, t-butylperoxy- pivalate and the like. Oil-soluble catalysts are preferred.

Thereafter, the reaction mixture is maintained at a temperature in the range of about 55° to 85°C., preferably, about 75° to 85°C., and most preferably, about 78° to 82°C., for a period of time sufficient to effect the desired polymerization and form the aqueous polymer solution necessary to stabilize the discrete microdroplets of the oil.

As the polymerization proceeds, the dispersed oil droplets become white and appear to precipitate in the aqueous medium, however, without coalescing. Generally, the observance of this white or milky color in the aqueous medium is an indication of completion of the process, which usually takes about 2 to 20 hours, preferably about 4 to 10 hours, and most preferably, about 6 to 8 hours. After completion of polymerization, the residual vinyl monomer content generally is less than about 0.1%, as measured by the iodine titration method.

The production of stable, discrete microdroplets of oil in the resulting aqueous polymer solution can be controlled by the viscosity of the aqueous polymer solution. For example, the viscosity of this medium can be increased by increasing the relative amount of vinyl monomer to oil in the original reaction mixture. By increasing the viscosity of the polymer solution, the proclivity to form a stable, homogeneous suspension of discrete microdroplets of oil throughout the entire medium is enhanced. On the other hand, reducing the viscosity of the medium by decreasing the amount of vinyl monomer in the initial mixture results in a more dilute concentration of polyvinyl polymer in the resultant mixture, which enhances the tendency to form a separate layer of discrete oil droplets.

Suitably, the ratio of monomer to oil used in the polymerization should be in the range of about 95/5 to 5/95, respectively, on a weight basis, preferably at least about 50/50. Most preferred is a range of about 90/10 to 70/30. As used herein, a "stable composition or suspension" means that the discrete oil microdroplets remain suspended in the aqueous polymer solution for at least seven days at ambient temperature.

The viscosity of the stabilized oil in water product, for example, polyvinylpyrrolidone polymer, which is obtained by in situ polymerization of vinylpyrrolidone monomer, suitably is in the range of about 3,000 to 100,000 cps, preferably about 4,000 to 60,000 cps(mPa.s), and most preferably, about 6,000 to 25,000 cps.

The diameter of the oil microdroplets obtained are observed to be in the range of about 0.1 to 450 micrometers, and usually are about 1 to 100 micrometers.

The microdroplets of silicone oil as produced in Example 1 are stabilized homogeneously throughout an aqueous polyvinylpyrrolidone solution made by in situ polymerization of vinylpyrrolidone in water.

Suitable surfactants for forming the stable oil dispersion herein include nonionic, synthetic anionic, amphoteric and zwitterion surfactants, preferably present at a level of about 0.01 to 10% by weight of the polymerization mixture. A mixture of nonionic surfactants are preferred, which are available as Emulphogene BC-610 and BC-840, and which have HLB values of about 11 and 14, respectively.

The surfactants useful in the conditioning hair care compositions of this invention can be present at a level of from about 1% to about 30%, most preferably from about 5% to about 25% of the composition. Surfactants useful in such compositions of the invention include anionic, nonionic, cationic, zwitterionic and amphoteric surfactants.

The conditioning hair care composition comprising (a) a stabilized silicone product obtained by in situ copolymerization of a water-soluble vinyl monomer, preferably vinylpyrrolidone, and a water-soluble acryl comonomer, preferably MAPTAC, in the presence of discrete microdroplets of a silicone oil in water, (b) a surfactant, and the balance being (c) water.

The hair and skin care cosmetic compositions in accordance with the present invention containing the discrete, cosmetically-active oil dispersion as defined above can be provided under different forms.

The hair and skin care cosmetic compositions according to the invention can contain the dispersion either as the principal active component or as an additive.

Moreover, these compositions generally contain at least one conventional adjuvant used in cosmetic compositions.

The hair and skin care cosmetic compositions can be provided in the form of aqueous, alcoholic or hydroalcoholic solutions, the alcohol being principally a lower alkanol such as ethanol or isopropanol, or in the form of a cream, a gel, an emulsion or even in the form of an aerosol packaged under pressure in an aerosol container together with a propellant.

The adjuvants generally provided in the cosmetic compositions according to the invention are, for example, perfumes, dyes, preservatives, sequesterants, thickening agents and the like.

The cosmetic compositions according to the invention are either compositions ready for use or concentrates which can be diluted before use.

The cosmetic compositions according to the invention are not limited to a particular concentration of the dispersion described above. However, generally, in the cosmetic compositions according to the invention, the concentration of the PVP-silicone oil dispersion is between 0.1 and 25 weight percent and preferably between 1 and 10 weight percent.

As has been indicated above, the dispersion of the cosmetically-active oil in the cosmetic composition of the invention imparts principally advantageous cosmetic characteristics when they are applied to the hair or skin of the user.

The hair and skin care cosmetic compositions in accordance with the invention are characterized by the fact that they contain stable, discrete microdroplets of a cosmetically-active oil in the form of a dispersion.

These cosmetic compositions for the hair and skin can be provided in the form of aqueous, alcoholic or hydroalcoholic solutions, the alcohol being either ethanol or isopropanol, preferably in the form of a cream, a mousse, a lotion, an oil, a water-in-oil emulsion or even in the form of a spray. In this latter case, the compositions are packaged in an aerosol container, under pressure, together with a propellant such as nitrogen, nitrous oxide, carbon dioxide, butane or even mixtures of such propellants.

As has been indicated above, the cosmetic composition according to this invention is preferably employed for the care or treatment of the hair or skin.

In skin use, these compositions facilitate the hydration of the skin and avoid its drying out. These compositions also impart to the skin excellent softness to the touch.

The cosmetic compositions for the skin are provided preferably in the form of lotions, creams, gels, emulsions, mousses or aqueous, alcoholic or hydroalcoholic solutions.

The adjuvants generally present in these cosmetic compositions are, for example, perfumes, dyes, preservatives, thickening agents, sequesterants, emulsifiers, solar filters and the like.

These compositions for the skin constitute principally treating creams or lotions for the hands, face or body, sunscreens, and cleansing lotions.

These compositions are prepared according to known methods.

For example, to obtain a cream, an aqueous phase containing in solution the dispersion and optionally other components or adjuvants is emulsified with an oily phase.

The oily phase can be constituted by various compounds such as, for example, paraffin oil, petrolatum oil, sweet almond oil, avocado oil, olive oil, esters of fatty acids such as glyceryl monostearate, ethyl or isopropyl palmitates, alkyl myristates such as propyl, butyl or cetyl myristates. Fatty alcohols such as cetyl alcohol or waxes such as beeswax can also be added.

Water is an essential component of the conditioning hair care compositions and generally comprises from about 20% to about 98% of the total composition.

The present compositions herein can contain a variety of other optional components suitable for rendering such compositions more acceptable. Such conventional optional ingredients are well known to those skilled in the art, e.g., pearlescent aids such as ethylene glycol distearate; preservatives such as benzyl alcohol, methyl paraben, propyl paraben and imidazolidinyl urea; thickeners and viscosity modifiers such as a diethanolamide of a long chain fatty acid (e.g., lauric diethanolamide), cocomonoethanol amide, dimethicone copolyols, guar gum, methyl cellulose starches and starch derivatives, fatty alcohols such as cetearyl alcohol, sodium chloride, sodium sulfate, polyvinyl alcohol, and ethyl alcohol; pH adjusting agents such as citric acid, lactic acid, sodium citrate, succinic acid, phosphoric acid, sodium hydroxide, sodium carbonate, etc.; coloring agents such as any of the FD&C or D&C dyes; hair oxidizing (bleaching) agents such as hydrogen peroxide, perborate and persulfate salts; hair reducing agents such as the thioglycolates; perfumes; and, sequestering agents such as disodium/tetrasodium ethylenediamine tetraacetate, polymer plasticizing agents such as glycerin and propylene glycol. Such agents generally are used individually at a level of from about 0.01% to about 10%, preferably from about 0.05% to about 5.0% by weight of the composition.

The pH of the present compositions is not critical and may be in the range of from about 3.5 to about 8.0.

As with all compositions, the present compositions should not contain components which unduly interfere with the performance of the compositions.

The invention will now be described with references to the following more particular examples.

EXAMPLE 1

The in situ polymerization process of the invention was carried out in a 1-liter laboratory reactor equipped with an overhead stirring motor, a metal anchor agitator, a nitrogen gas inlet tube, a water condenser connected to a bubbler, a temperature probe connected to a temperature controller and associated with a heating mantle, and a dropping funnel.

The reactor first was purged with nitrogen and charged with 400 g. of distilled water and 10 g. of Dimethicone oil having a viscosity of 100 cs. The oil-water then mixture was agitated vigorously at 350 rpm under nitrogen for 30 minutes whereupon the oil was dispersed as transparent, discrete microdroplets in the aqueous medium. The dispersion then was heated to 80°C. and 0.25 g. of di-tert-butylperoctoate was added. At this point, the mixture was maintained for 30 minutes with continuous stirring whereafter 90 g. of vinylpyrrolidone and an additional 0.25 g. of di-tert-butylperoctoate was added at one time while maintaining a nitrogen flow of 15 ml/min. After about 10-15 minutes, an exotherm was observed and the temperature increased to 86°C. The transparent, spherical droplets of oil became opaque. The the temperature was reduced to 80°C. and polymerization was continued for 6-8 hours with stirring. During this period, the dispersion became milky and the droplets became completely invisible. Polymerization was considered complete when the measured residual monomer content was less than 0.1%.

The composition obtained was a stable, homogeneous dispersion of microdroplets of Dimethicone oil stabilized in an aqueous polyvinylpyrrolidone solution. Upon exerting only slight pressure on the microdroplets, the silicone oil was observed to ooze out. However, the composition was quite stable for many months at room temperature, and for an extended period at the elevated temperature of 45° to 54°C.

EXAMPLES 2-3

The procedure of Example 1 was repeated using weight ratios of 80 g. of vinylpyrrolidone to 20 g. of Dimethicone oil (Example 2), and 70 g. of vinylpyrrolidone to 30 g. of Dimethicone oil (Example 3). Similar results to Example 1 were obtained in these runs.

EXAMPLE 4

The procedure of Example 1 was followed using a weight ratio of 20 g. of vinylpyrrolidone and 80 g. of Dimethicone oil. The resultant composition was not as viscous as in Example 1. The microdroplets obtained remained in discrete form, however, without coalescence, but settled to the bottom of the solution as a separate layer.

EXAMPLE 5

The procedure of Example 1 was followed using a weight ratio of 135 g. of vinylpyrrolidone to 15 g. of Dimethicone oil in 600 ml. of water. The results were substantially the same as obtained in Example 1.

EXAMPLE 6

The procedure of Example 1 was followed using a weight ratio of 135 g. of vinylpyrrolidone to 15 g. of a Dimethicone oil having a viscosity of 1,000 cs (mol. wt. of 28,000). The mixture was agitated at 700 rpm to produce a stable dispersion of the viscous silicone oil droplets in the aqueous polymer solution.

EXAMPLE 7

A pilot plant run was carried out in a 30 gal. (113,6ℓ). reactor using two wide span turbine agitators having pitched and flat blades set at 200 rpm. 10,790 g. of vinylpyrrolidone, 1205 g. of Dimethicone oil, 100 cs, 48,225 g. of water, 120 g. of di-t-butylperoctoate, and 317 g. of Germaben[®] preservative were used in this run. After 6 hours, polymerization was complete and a stable, homogeneous, milky aqueous dispersion of discrete, coated silicone oil droplets was obtained which dispersion remained in discrete and suspended form throughout the composition. The composition also was stable for an extended period of time.

EXAMPLE 8

The procedure of Example 1 was followed using 102 g. of vinylpyrrolidone, 11 g. of Dimethicone, 100 cs, 36 g. of a 50% aqueous solution of methacrylamidopropyltrimethylammonium chloride, 462 g. of water, 0.1 g. of tetrasodium pyrophosphate, and 0.60 g. of di-tert-butylperoctoate. A stable, homogeneous composition was obtained having a residual VP content of only 0.01%.

EXAMPLE 9

The procedure of Example 1 was followed using 90 g. of vinylpyrrolidone, 10 g. of Dimethicone oil, 100 cs, 400 g.

of water and 0.75 g. of Lupersol 11. The results were similar to those obtained in Example 4.

EXAMPLE 10

The procedure of Example 1 was followed 90 g. of vinylpyrrolidone, 10 g. of light mineral oil having a density of 0.838 g/ml, 400 g. of water and 0.75 g. of Lupersol 11. The results were similar to Example 1.

EXAMPLE 11

The procedure of Example 10 was followed using 10 g. of heavy mineral oil having a density of 0.862 g/ml. The results were similar to Example 10.

CONTROL EXPERIMENTS C-1 TO C-6

Various blends of PVP polymer (K-90) and silicone oil were agitated vigorously and allowed to stand at room temperature for a day. Two layers were produced. The upper layer was a transparent, homogeneous liquid layer which contained silicone oil. The lower layer was an aqueous layer containing dissolved PVP. Discrete droplets of silicone oil were not observed in either layer. The results are presented as Examples C-1 to C-7 in Table II.

EXAMPLE 12

Film Formation

The composition of Example 1 was spread onto metal and glass substrates as films. The coated substrate was dried in vacuo at about 50°C. for about 24 hours to produce a homogeneous, thick, opaque, glassy film.

The results of these experiments are summarized in Tables I to IV below.

TABLE I

| Ex. No. | Monomer | Amt (g) | Silicone Oil | Amt (g) | Viscosity (cs)(mm$^2$.s$^{-1}$) | MW |
|---|---|---|---|---|---|---|
| 1 | VP | 90 | DM | 10 | 100 | 5970 |
| 2 | VP | 80 | DM | 20 | 100 | 5970 |
| 3 | VP | 70 | DM | 30 | 100 | 5970 |
| 4 | VP | 20 | DM | 80 | 100 | 5970 |
| 5 | VP | 135 | DM | 15 | 100 | 5970 |
| 6 | VP | 135 | DM | 15 | 1000 | 28,000 |
| 7* | VP | 10,790 | DM | 1205 | 100 | 5970 |
| 8 | VP | 102 | DM | 11 | 100 | 5970 |
| 9 | VP | 90 | DM | 10 | 100 | 5970 |
| 10 | VP | 90 | MO | 10 | | |
| 11 | VP | 90 | MO | 10 | | |

* Pilot plant run

TABLE I-A

| Ex. No. | Comonomer | Amt (g) | Medium | Amt (g) | Initiator | Amt (g) | Agitation (rpm) |
|---|---|---|---|---|---|---|---|
| 1 | -- | -- | Water | 400 | TBP | 0.75 | 350 |
| 2 | -- | -- | Water | 400 | TBP | 0.75 | 350 |
| 3 | -- | -- | Water | 400 | TBP | 0.75 | 350 |
| 4 | -- | -- | Water | 400 | TBP | 0.75 | 350 |
| 5 | -- | -- | Water | 600 | TBP | 0.76 | 350 |
| 6 | -- | -- | Water | 600 | TBP | 0.76 | 700 |
| 7* | -- | -- | Water | 48,225 | TBP | 120 | 200 |
| 8 | MAPTAC | 18 | Water | 462 | TBP | 0.60 | 350 |
| 9 | -- | -- | Water | 400 | TBPP | 0.75 | 350 |
| 10 | -- | -- | Water | 400 | TBP | 0.75 | 350 |
| 11 | -- | -- | Water | 400 | TBP | 0.75 | 350 |

* Pilot plant run

TABLE II

| Ex. No. | Polymer | % (lb)* | | Silicone Oil | % (lb)* | | Viscosity (cs)(mm$^2$.s$^{-1}$) | M.W. |
|---|---|---|---|---|---|---|---|---|
| C-1 | PVP | 90 | 24.34 | DM | 10 | 2.76 | 100 | 5970 |
| C-2 | PVP | 80 | 21.52 | DM | 20 | 5.38 | 100 | --- |
| C-3 | PVP | 70 | 18.83 | DM | 30 | 8.07 | 100 | --- |
| C-4 | PVP | 80 | 21.49 | DM | 20 | 5.37 | 1000 | 28,000 |
| C-5 | PVP | 70 | 21.49 | DM | 30 | 5.37 | 1000 | 28,000 |
| C-6 | PVP | 80 | 21.49 | DM | 20 | 5.37 | 12,500 | 67,700 |
| C-7 | PVP | 70 | 21.49 | DM | 30 | 5.37 | 12,500 | 67,700 |

* 1 lb = 0.453 kg.

TABLE II-A

| Ex. No. | Medium | Amt (lb)* | Agitation (rpm) |
|---|---|---|---|
| C-1 | Water | 110.90 | 220 |
| C-2 | Water | 106.91 | 220 |
| C-3 | Water | 106.91 | 220 |
| C-4 | Water | 106.82 | 220 |
| C-5 | Water | 106.82 | 220 |
| C-6 | Water | 106.82 | 220 |
| C-7 | Water | 106.82 | 220 |

* 1 lb = 0.453 kg.

TABLE III

| Ex. No. | % Solids | Brookfield Viscosity (cps)(mPa.s) | Diameter of Microspheres (micrometers) | | Form of Composition |
|---------|----------|-----------------------------------|------|-------|---------------------|
| | | | Mean | Range | |
| 1 | 19.7 | 7,200 | - | - | stable, homogeneous, milky dispersion of discrete microspherical droplets of silicone oil |
| 2 | 22.0 | 24,400 | | 1-14 | coated by polyvinylpyrrolidone polymer |
| 3 | 21.1 | 17,300 | | 1-17 | |
| 4 | 20.0 | --- | | --- | |
| 5 | | | | | |
| 6 | 20.6 | 10,200 | 56 | | |
| 7 | 20.2 | 8,900 | 80 | 3-54 | |
| 8 | 30.3 | 11,300 | - | --- | |
| 9 | 20.2 | 7,200 | - | --- | |
| 10 | 20.45 | 4,770 | - | 0.4-13 | as in Ex. 1 |
| 11 | 21.00 | 3,180 | - | 0.5-29 | as in Ex. 1 |

TABLE IV

| Ex. No. | Brookfield Viscosity (cps)(mPa.s) | | Range of Diameters of Microspheres (microns) |
|---------|-----------------------------------|---|----------------------------------------------|
| C-1 | 8,600 | 9 | 2-45 |
| C-2 | 7,970 | 8 | 3-76 |
| C-3 | 5,310 | 7 | 3-55 |
| C-4 | 5,960 | 7 | 3-109 |
| C-5 | 9,140 | 8 | 3-174 |
| C-6 | 9,860 | 8 | 5-43 |
| C-7 | 6,152 | 7 | 3-77 |

EXAMPLE 13

A mixture of 15.3 g. of polydimethylsiloxane and 1.6 g. of Emulphogene BC 610 (Rhone-Poulenc Inc.), a nonionic surfactant, having an HLB value of 11, was formed with the aid of a homogenizer. Then 2.4 g. of Emulphogene BC 840, another nonionic surfactant, having an HLB value of 14, was admixed with further stirring. Thereafter 50 g. of distilled, deionized water was slowly introduced into the mixture in increments of 10 ml. After each addition, the aqueous mixture was homogenized. A creamy, white emulsion was formed. Then an additional 550 g. of water was added slowly while stirring vigorously.

The resulting emulsion was transferred to a polymerization kettle equipped with a dropping funnel, water condenser, a $N_2$ inlet, an overhead stirring motor and a metal stirrer. The mixture was stirred at 200 rpm, purged with $N_2$ for 1 hour, 0.506 g. of Trigonox 21 was added, and the reaction mixture was heated to 82°C. After 20 minutes, a solution of 135 g. of freshly distilled vinylpyrrolidone and an additional amount of 0.301 g. of Trigonox 21 was added through the dropping funnel over 30 minutes. After the addition was completed, an exotherm was observed which raised the temperature of the reactants to 87°C. Polymerization was carried out at 82°C. and continued until the unreacted vinylpyrro-

9

lidone monomer content dropped to 0.53%. The resulting product was a white, homogeneous, aqueous polymer composition containing dispersed microspheres of PVP in which the siloxane was entrapped. The microspheres remained uniform upon standing for an extended period of time and did not coalesce.

EXAMPLES 14-18

The procedure of Example 13 was followed using polydimethylsiloxanes having a kinematic viscosity in the range of 100 to 100,000 cs, at a pH in the range of about 4.0 to 7.5. White, liquid, homogeneous products were obtained which remained uniform upon standing.

TABLE 1

| Ex. No. | Monomer | Amt (g) | Silicone Oil | Amt (g) | Viscosity (cs)(mm$^2$.s$^{-1}$) | MW |
|---------|---------|---------|--------------|---------|-------------------|----|
| 13-140 | VP | 135 | DM | 15.3 | 12,500 | 67,700 |
| 14-142 | VP | 135 | DM | 16.0 | 100,000 | 139,000 |
| 15-147 | VP | 135 | DM | 15.0 | 12,500 | 67,700 |
| 16-151 | VP | 135 | DM | 15.0 | 100,000 | 139,000 |
| 17-143 | VP | 135 | DM | 15.0 | 100 | 5,970 |
| 18-144 | VP | 135 | DM | 15.0 | 1,000 | 28,000 |

TABLE 2

| Ex. No. | Surfactant | Amt (g) | HLB | Medium | Amt (g) | pH | Initiator | Amt (g) | Agitation (rpm) |
|---------|-----------|---------|-----|--------|---------|----|-----------|---------|-----------------|
| 13-140 | EPG BC-610 | 1.6 | 11.4 | Water | 600 | 4.0 | TBP | 0.81 | 200 |
| | EPG BC-840 | 2.1 | 15.4 | | | | | | |
| 14-142 | BC 610/840 | 2.5/9.9 | -- | Water | 600 | 4.0 | TBP | 0.76 | 200 |
| 15-147 | BC 610/840 | 1.7/2.5 | -- | Water | 600 | 7.5 | TBP | 0.76 | 200 |
| 16-151 | BC 610/840 | 2.5/9.10 | -- | Water | 600 | 7.5 | TBP | 0.78 | 200 |
| 17-143 | BC 610/840 | 1.7/2.5 | -- | Water | 600 | 7.5 | TBP | 0.77 | 200 |
| 18-144 | BC 610/840 | 1.7/2.5 | -- | Water | 600 | 7.5 | TBP | 0.77 | 200 |

TABLE 3

| Ex. No. | % Solids | Brookfield Viscosity (cps)(mPa.s) |
|---------|----------|-----------------------------------|
| 13-140 | 19.2 | 220 |
| 14-142 | 19.7 | 216 |
| 15-147 | 20.0 | 8,170 |
| 16-151 | 20.0 | 7,300 |
| 17-143 | 20.0 | >8,000 |
| 18-144 | 20.0 | 10,160 |

The following examples are representative of the conditioning hair care compositions of the present invention.

TABLE A

| CONDITIONING SHAMPOO COMPOSITION | | | |
|---|---|---|---|
| Components Essential Components | Concentration (%) by Weight | | |
| | Suitable | Preferred | Optimum |
| (a) Stabilized Silicone Product (of Exs 1-9, 20% active) | 0.25-25 | 1-10 | 2.5 |
| (b) Surfactant | 4-25 | 7-20 | 15 |
| (c) Water | qs | qs | qs |

TABLE B

| PROPERTIES OF CONDITIONING SHAMPOO COMPOSITION OF INVENTION | | | |
|---|---|---|---|
| | Composition | | |
| | Suitable | Preferred | Optimum |
| pH | 4-7.5 | 5-7 | 6 |
| Viscosity, cps(mPa.s) | 2,000-15,000 | 3,000-8,000 | 5,000 |

The following are specific examples representative of the conditioning shampoo composition of the present invention.

TABLE C

| Component | | Weight % | | |
|---|---|---|---|---|
| | | I | II | III |
| | VP/Silicone | | | |
| PVP-Silicone Product (20% active) | (90/10) Ex 1 | 15.0 | 0.0 | 0.0 |
| | (80/20) Ex 2 | 0.0 | 15.0 | 0.0 |
| | (70/30) Ex 3 | 0.0 | 0.0 | 15.0 |
| Ammonium lauryl sulfate (30% active) | | 30.9 | 30.0 | 30.5 |
| Lauric Diethanolamide (100% active) | | 5.0 | 5.0 | 5.0 |
| Preservative | | 0.5 | 0.5 | 0.5 |
| Fragrance | | 0.2 | 0.2 | 0.2 |
| Water | | qs | qs | qs |

The conditioning shampoo compositions of the invention exhibit excellent properties in actual use on hair including effective curl retention, enhanced hair stiffness, and advantageous curl snap, in direct comparative testing with other related products having silicone dispersed therein.

| AEROSOL CONDITIONING AND STYLING MOUSSE | | | |
|---|---|---|---|
| Essential | Suitable | Preferred | Optimum |
| PVP-Silicone (90/10, Ex. 1) (20% Active) | 0.5-10 | 1-5 | 3.0 |
| Resin (Gaffix® VC-713, GAF) | 1-10 | 2-8 | 5.0 |
| Surfactant (non-ionic, nonoxynol-9 or sodium cocoylisethionate) | 0.1-5 | 0.2-1 | 0.5 |
| Water | qs | qs | qs |
| Propellant (A-46, isobutane/propane) | 5-25 | 10-20 | 15.0 |

The following is a specific example of the conditioning mousse of the invention.

| Ingredient | % by weight |
|---|---|
| PVP-Silicone (90/10, Ex. 1) | 3.0 |
| Vinylpyrrolidone/dimethylaminoethyl methacrylate quaternized w/diethylsulfate | 5.00 |
| Oleth-20 | 0.5 |
| Fragrance | 0.25 |
| Propellant A-46 | 15.0 |
| DM DM Hydantoin | 0.25 |
| Deionized water | 76.0 |
| | 100.00 |

The expansion properties of the foam in the mousse composition is enhanced by the presence of the PVP-silicone therein. In addition, the texture and feel of the foam is smoother with the PVP-silicone present. Furthermore the mousse is effective for mending split ends of hair.

| CONDITIONER COMPOSITION | | | |
|---|---|---|---|
| | Suitable | Preferred | Optimum |
| PVP-Silicone (90/10, Ex. 1) (20% solids) | 0.5-10 | 1-5 | 2.5 |
| Emulsifier/thickener | 1-10 | 2-7 | 3.5 |
| Cationic surfactant | 0-10 | 2-5 | 3.0 |
| Water | qs | qs | qs |

The conditioner composition is effective to mend split ends of hair.

| Ingredient | % by weight |
|---|---|
| Emulsifying wax | 3.5 |
| Stearyl alcohol + Ceteareth-20 | 1.5 |
| Glycol stearate | 0.5 |
| PVP/Silicone (90/10, Ex. 1) | 2.5 |
| Laneth-16 + Ceteth-16 + Oleth-16 + Steareth-16 | 0.3 |
| Methylparaben | 0.2 |
| Propylparaben | 0.1 |
| Water | qs |

In combination with the above composition, the addition of stearyl dimethylbenzyl ammonium chloride in the amount of 2-10% by weight of the composition as a cationic surfactant improves the detangling properties of the composition.

The following examples are representative of other cosmetic compositions of the present invention.

| LOTION FOR DRY SKIN | | | |
|---|---|---|---|
| Essential | Suitable | Preferred | Optimum |
| | | (% by Wt.) | |
| PVP-Siicone (90/10, Ex. 1) (20% solids) | 0.5-10 | 1-5 | 3.0 |
| Stabilizer | 0.05-0.5 | 0.1-0.3 | 0.15 |
| Emulsifier | 1-10 | 2-8 | 3.0 |
| Soap | 1-10 | 2-8 | 3.0 |
| Wax | 0-10 | 3-8 | 5.0 |
| Neutralizer | 0.2-1 | 0.4-1 | 1.0 |
| Water | qs | qs | qs |

The following is a specific example of the lotion for dry skin composition of the invention.

| Ingredient | % by weight |
|---|---|
| Distilled water | 85.10 |
| Polyacrylic acid (crosslinked) | 0.15 |
| Stearic acid, XXX | 3.00 |
| Mineral oil, 70 cts | 2.00 |
| Emulsifying wax | 3.00 |
| PVP-Silicone (90,10 Ex. 1) (20% solids) | 3.00 |
| Oleth-20 | 1.50 |
| Triethanolamine | 1.00 |
| Methylparaben/propylparaben | 1.00 |
| Fragrance | 0.25 |
| | 100.00 |

The presence of the PVP-silicone in the lotion enhances the smoothness, softness and silky feel of the skin.

The following are other representative skin care compositions in which the presence of PVP-silicone is advantageous for the user.

| BATH PREPARATION | |
|---|---|
| PVP-Silicone (Ex. 1) | 3.0 |
| ammonium nonoynol-4-sulfate | 30.0 |
| sodium cocoyl isothionate | 10.0 |
| cocamidopropyl hydroxysultaine | 10.0 |
| cocamide diethanolamide | 6.0 |
| sodium methyl cocyl taurate | 20.0 |
| aloe vera gel | 1.0 |
| coconut oil | 1.0 |
| glycol stearate | 1.0 |
| deionized water | qs |
| preservative | qs |
| colorant | qs |

| SUSCREEN LOTION (1) | |
|---|---|
| PVP-Silicone (Ex. 1) | 2.5 |
| sorbitol | 6.0 |
| propylparaben | 1.0 |
| glyceryl stearate | 2.4 |
| stearic acid | 1.5 |
| octyl dimethyl PABA | 7.5 |
| benzophenone-3 | 2.5 |
| lanolin | 2.5 |
| methylparaben | 0.2 |
| deionized water | qs |

| SKIN CLEANSER FOR OILY SKIN | |
|---|---|
| PVP-Silicone (Ex. 1) | 2.5 |
| propylene glycol | 5.0 |
| hydroxyethylcellulose | 0.9 |
| sodium laureth sulfate (30% active) | 15.0 |
| preservative | 0.75 |
| germacidal agent | 6.0 |
| water | qs |

| MOISTURIZING LOTION | |
|---|---|
| PVP-Silicone (Ex. 1) | 2.0 |
| mineral oil 70 CTS | 2.0 |
| stearic acid | 3.0 |
| emulsifying wax | 3.0 |
| Dimethicone* 200 CTS | 1.5 |
| Carbomer 934** | 0.15 |
| Oleth-20*** | 1.0 |
| triethanolamine 98% | 1.0 |
| deionized water | qs |
| preservative | qs |
| fragrance | qs |

* a mixture of methylated siloxane
polymers end-blocked with trime-
thyl siloxy units (dimethylpolysi-
loxane)
** cross-linked polymer of acrylic
acid
*** PEG ether of oleyl alcohol

| CATIONIC MOUSSE HAND/BODY LOTION (Used 85 Parts of the following formula to 15 parts propellant A-46) | |
|---|---|
| PVP-Silicone (Ex. 1) | 0.50 |
| acetylated polyoxyethylene lanolin | 2.00 |
| ethoxylated lanolin alcohols | 1.00 |
| glyceryl stearate, self-emulsifying | 5.50 |
| cetyl alcohol | 1.50 |
| mineral oil, 70 CTS | 1.50 |
| stearyl alcohol | 1.50 |
| glycerin | 3.00 |
| isopropyl myristate | 4.00 |
| dimethicone, 100 CTS | 2.00 |
| water | qs |
| preservative | qs |
| fragrance | qs |

| AFTER SHAVE BALM | |
|---|---|
| PVP-Silicone (Ex. 1) | 1.00 |
| Carbomer 941 | 0.20 |
| tetrasodium ethylene diamine tetracetic acid | 0.10 |
| cetearyl alcohol* polyethylene glycol ether of cetearyl alcohol | 2.50 |
| isopropyl myristate | 1.00 |
| Oleth-20 | 1.00 |
| methyl gluceth 20 | 2.00 |
| triethanolamine, 98% | 0.20 |
| propylene glycol | 3.00 |
| SDA denatured alcohol | 7.50 |
| PVP/dimethylaminoethyl methacrylate | 7.00 |
| fragrance | 1.00 |
| distilled water | qs |

\* 50/50 mixture of cetyl and stearyl alcohols

| SELF-HEATING AEROSOL SHAVING CREAM (Used dual dispensing valve containing 30 ml of 3% hydrogen peroxide) | |
|---|---|
| PVP-Silicone (Ex. 1) | 2.00 |
| stripped coconut fatty acid | 1.10 |
| sorbitol | 10.00 |
| stearic acid | 4.20 |
| PEG-40 soritan peroleate | 2.00 |
| triethanolamine | 3.00 |
| potassium hydroxide | 1.00 |
| potassium sulfite | 9.00 |
| fragrance | 0.80 |
| butyrated hydroxy toluene (BHT) | 0.01 |
| butyrated hydroxy anisole (BHA) | 0.01 |
| deionized water | qs |

| ANTIPERSPIRANT STICK | |
|---|---|
| PVP-Silicone (Ex. 1) | 10 |
| stearyl alcohol | 20 |
| cyclomethicone | 40 |
| aluminum chlorhydrate | 20 |
| acetylated sucrose distearate | 2.5 |
| talc | 1.5 |
| amorphous fused silica | 2 |
| myristyl ether PM-3 | 3 |
| polyoxyethylene glycol stearate | 1 |

Similar cosmetic products for the care of hair and skin were prepared using dispersions containing MAPTAC comonomer according to Example 8 as a 30% active dispersion in which the weight ratio PVP-MAPTAC-silicone was 102/18/11.

**Claims**

1.  A method for stabilizing an oil such as a cosmetically-active material, for example a silicone oil having a viscosity of about 5 to 600,000 cs (mm$^2$.s$^{-1}$), in water which is characterized by dispersing the oil in water, optionally with a surfactant, as microdroplets, adding a water-soluble vinyl monomer, such as vinyl pyrrolidone, optionally with a comonomer, such as an acryl comonomer, and a free radical polymerization initiator thereto, and in situ polymerizing said monomer whereby the oil microdroplets are stabilized as a homogeneous or heterogeneous distributed dispersion in the resulting polymer solution.

2.  A method according to claim 1 wherein said oil is a silicone having a viscosity between about 100 and 100,000 cs (mm$^2$.s$^{-1}$).

3.  A method according to claim 1 wherein the weight ratio of the vinylpyrrolidone monomer to oil in the polymerization mixture is about 95:5 to 5:95, respectively, on a weight basis.

4.  A method according to claim 1 wherein the Brookfield viscosity of the stabilized oil in water product obtained upon in situ polymerization is about 3,000 to 100,000 cps (mPa.s).

5.  A composition comprising discrete microdroplets of an oil stabilized in an aqueous polymer solution of an in situ polymerized water-soluble vinyl monomer according to claim 1.

6.  A composition according to claim 5 which is a conditioning hair care composition comprising

    (a) a stabilized silicone product obtained by in situ polymerization of a water-soluble vinyl monomer in the presence of discrete microdroplets of a silicone in water,
    (b) a surfactant, and the balance being
    (c) water.

7.  A conditioning hair care composition according to claim 6 comprising about 0.25 to 25% by weight of (a), about 4 to 25% by weight of (b), the balance being water.

8.  A conditioning hair care composition according to claim 6 which is an aerosol conditioning mousse.

9.  A composition according to claim 5 which is a skin care composition containing about 0.1 to 25 weight percent of a stabilized cosmetically-active product obtained by in situ polymerization of a water-soluble vinyl monomer in the presence of discrete microdroplets of a cosmetically-active oil in water.

10. A composition according to claim 5 which is a hair or skin care composition containing about 0.1 to 25 weight percent of a stabilized cosmetically-active product obtained according to claim 1 in the presence of an acryl comonomer microdroplets of a cosmetically-active oil in water.

11. Use of a water-soluble vinyl polymer, obtained by polymerisation in situ in a dispersion of oil in water, to stabilise the oil as microdroplets dispersed in the resulting polymer solution.

**Patentansprüche**

1. Verfahren zum Stabilisieren eines Öls, wie z.B. eines kosmetisch aktiven Materials, beispielsweise eines Silikonöls mit einer Viskosität von etwa 5 bis 600.000 cSt (mm$^2 \cdot$s$^{-1}$), in Wasser, gekennzeichnet durch Dispergieren des Öls in Wasser, gegebenenfalls mit einem Tensid, als Mikrotröpfchen, Zusetzen eines wasserlöslichen Vinylmonomers, wie z.B. Vinylpyrrolidon, gegebenenfalls mit einem Comonomer, wie z.B. einem Acrylcomonomer, und eines Freiradikal-Polymerisationsinitiators dazu und durch in-situ Polymerisieren des Monomers, wodurch die Öl-Mikrotröpfchen als homogene oder heterogene, verteilte Dispersion in der resultierenden Polymerlösung stabilisiert werden.

2. Verfahren nach Anspruch 1, worin das Öl ein Silikon mit einer Viskosität zwischen etwa 100 und 100.000 cSt (mm$^2 \cdot$s$^{-1}$) ist.

3. Verfahren nach Anspruch 1, worin das Gewichtsverhältnis des Vinylpyrrolidonmonomers zum Öl im Polymerisationsgemisch jeweils etwa 95:5 bis 5:95, bezogen auf das Gewicht, beträgt.

4. Verfahren nach Anspruch 1, worin die Brookfield-Viskosität des durch in-situ Polymerisation erhaltenen, stabilisierten Öl-in-Wasser-Produkts etwa 3.000 bis 100.000 cp (mPa$\cdot$s) beträgt.

5. Zusammensetzung, umfassend diskrete Mikrotröpfchen eines Öls, stabilisiert nach Anspruch 1 in einer wäßrigen Polymerlösung eines in-situ polymerisierten, wasserlöslichen Vinylmonomers.

6. Zusammensetzung nach Anspruch 5, das eine konditionierende Haarpflegezusammensetzung ist, umfassend:

    (a) ein stabilisiertes Silikonprodukt, erhalten durch in-situ Polymerisation eines wasserlöslichen Vinylmonomers in Gegenwart diskreter Mikrotröpfchen eines Silikons in Wasser,
    (b) ein Tensid, wobei der Rest aus
    (c) Wasser besteht.

7. Konditionierende Haarpflegezusammensetzung nach Anspruch 6, umfassend etwa 0,25 bis 25 Gew.-% (a), etwa 4 bis 25 Gew.-% (b), wobei der Rest aus Wasser besteht.

8. Konditionierende Haarpflegezusammensetzung nach Anspruch 6, die ein konditionierender Aerosolschaum ist.

9. Zusammensetzung nach Anspruch 5, die eine Hautpflegezusammensetzung ist, umfassend etwa 0,1 bis 25 Gew.-% eines stabilisierten, kosmetisch aktiven Produkts, erhalten durch in-situ Polymerisation eines wasserlöslichen Vinylmonomers in Gegenwart diskreter Mikrotröpfchen eines kosmetisch aktiven Öls in Wasser.

10. Zusammensetzung nach Anspruch 5, die eine Haar- oder Hautpflegezusammensetzung ist, umfassend etwa 0,1 bis 25 Gew.-% eines stabilisierten, kosmetisch aktiven Produkts, erhalten nach Anspruch 1 in Gegenwart von Acrylcomonomer-Mikrotröpfchen eines kosmetisch aktiven Öls in Wasser.

11. Verwendung eines wasserlöslichen Vinylpolymers, erhalten durch in-situ Polymerisation in einer Dispersion von Öl in Wasser, um das Öl als in der resultierenden Polymerlösung dispergierte Mikrotröpfchen zu stabilisieren.

**Revendications**

1. Méthode de stabilisation d'une huile telle qu'une matière cosmétiquement active, par exemple une huile de silicone ayant une viscosité d'environ 5 à 600 000 cs (mm$^2$.s$^{-1}$), dans l'eau qui est caractérisée par la dispersion de l'huile dans l'eau, facultativement avec un agent tensio-actif, sous la forme de microgouttelettes, l'addition d'un monomère vinylique soluble dans l'eau tel que la vinyl pyrrolidone, facultativement avec un comonomère tel qu'un comonomère acrylique et un initiateur de polymérisation de radicaux libres et la polymérisation in situ dudit monomère ce par quoi les microgouttelettes d'huile sont stabilisées sous la forme d'une dispersion distribuée homogène ou hétérogène dans la solution résultante du polymère.

**2.** Méthode selon la revendication 1 où ladite huile est une silicone ayant une viscosité entre environ 100 et 100 000 cs (mm$^2$.s$^{-1}$).

**3.** Méthode selon la revendication 1 où le rapport pondéral du monomère de vinylpyrrolidone à l'huile dans le mélange de polymérisation est d'environ 95:5 à 5:95, respectivement, sur une base pondérale.

**4.** Méthode selon la revendication 1 où la viscosité Brookfield du produit stabilisé de l'huile dans l'eau que l'on obtient lors d'une polymérisation in situ est d'environ 3 000 à 100 000 cps (mPa.s).

**5.** Composition comprenant des microgouttelettes discrètes d'une huile stabilisée dans une solution aqueuse d'un polymère d'un monomère vinylique soluble dans l'eau polymérisé in situ selon la revendication 1.

**6.** Composition selon la revendication 5 qui est une composition de conditionnement pour le soin des cheveux comprenant

(a) un produit stabilisé de silicone obtenu par polymérisation in situ d'un monomère vinylique soluble dans l'eau en présence de microgouttelettes discrètes d'une silicone dans l'eau,
(b) un agent tensio-actif, le reste étant
(c) de l'eau.

**7.** Composition de conditionnement pour le soin des cheveux selon la revendication 6 comprenant environ 0,25 à 25% en poids de (a), environ 4 à 25% en poids de (b), le reste étant de l'eau.

**8.** Composition de conditionnement pour le soin des cheveux selon la revendication 6 qui est une mousse de conditionnement en aérosol.

**9.** Composition selon la revendication 5 qui est une composition de soin de la peau contenant environ 0,1 à 25 pour cent en poids d'un produit stabilisé cosmétiquement actif obtenu par polymérisation in situ d'un monomère vinylique soluble dans l'eau en présence de microgouttelettes discrètes d'une huile cosmétiquement active dans l'eau.

**10.** Composition selon la revendication 5 qui est une composition de soin des cheveux ou de la peau contenant environ 0,1 à 25 pour cent en poids d'un produit stabilisé cosmétiquement actif obtenu selon la revendication 1 en présence de microgouttelettes d'un comonomère acrylique d'une huile cosmétiquement active dans l'eau.

**11.** Utilisation d'un polymère vinylique soluble dans l'eau obtenu par polymérisation in situ dans une dispersion d'huile dans l'eau, pour stabiliser l'huile sous la forme de microgouttelettes dispersées dans la solution résultante du polymère.